Europäisches Patentamt

European Patent Office

Office européen des brevets

(19)

(11) **EP 1 251 178 A1**

(12) **EUROPEAN PATENT APPLICATION**
published in accordance with Art. 158(3) EPC

(43) Date of publication:
**23.10.2002 Bulletin 2002/43**

(51) Int Cl.[7]: **C12N 15/57**, C07K 14/745

(21) Application number: **00982867.4**

(22) Date of filing: **18.12.2000**

(86) International application number:
**PCT/CN00/00589**

(87) International publication number:
**WO 01/046441 (28.06.2001 Gazette 2001/26)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **22.12.1999 CN 99125695**

(71) Applicant: **Biowindow Gene Development Inc. Shanghai**
**Shanghai 200092 (CN)**

(72) Inventors:
• **MAO, Yumin**
**Shanghai 200092 (CN)**
• **XIE, Yi**
**Shanghai 200092 (CN)**

(74) Representative: **Stark, Amanda Jane et al**
**Abel & Imray,**
**20 Red Lion Street**
**London WC1R 4PQ (GB)**

(54) **NOVEL POLYPEPTIDE-HUMAN SHC PROTEIN 43 AND POLYNUCLEOTIDE ENCODING IT**

(57) The invention concerns human SHC protein 43 and polynucleotide encoding it. The invention also concerns the process of producing the polypeptide by recombinant DNA technique. The methods for treating many diseases e.g. malignant tumor, hemopathy, infection of HIV, immunological diseases and a variety of inflammations utilizing the polypeptide are disclosed. The invention discloses the antagonist against the polynucleotide and therapeutics thereof. The invention also discloses the uses of the polynucleotide, which encodes human SHC protein 43.

EP 1 251 178 A1

**Description**

Field of Invention

The invention relates to the field of biotechnology. In particular, the invention relates to a novel polypeptide, human SHC protein 43, and a polynucleotide sequence encoding said polypeptide. The invention also relates to the method for the preparation and use of said polynucleotide and polypeptide.

Technical Background

A way to transduce the regulation signal of gene expression is by a series of phosphorylation cascade reactions, which is triggered by activation of some receptor Tyrosine kinases after they are bound by corresponding growth factors. Activated receptor Tyrosine kinases phosphorylate certain Tyrosine residues of their target proteins to activate them, and the phosphorylation-activated proteins come to phosphorylate and activate their target proteins until the signal finally arrives into the nucleus. The characteristic of this pathway is to fulfill transmembrane signal transduction without the aid of G protein, but only by activation of the Tyrosine kinase activity of the receptors themselves. So far, there are eight kinds of growth factor receptors found as to belong to this kind of signaling pathway: receptor of epidermal growth factor, receptor of platelet growth factor, receptor of liver cell growth factor, receptor of nerve growth factor, receptor of vascular endothelial growth factor, receptor of IDGF, receptor of insulin, and receptor of macrophage colony stimulating factor.

Current opinion on the mechanism how binding of growth factors to their receptors will activate Tyrosine kinase activity of receptor intracellular domains is that conformation change of the extracellular domain of a single growth factor receptor caused by ligand binding will trigger the dimerization of the receptor, and so Tyrosine residues located in their intracellular domain become closer and autophosphorylate each other. Result of autophosphorylation is the activation of receptor Tyrosine kinase.

Activated receptors can bind many cytoplasm located signaling proteins containing SH2 domain, and activate them by phosphorylation of their certain Tyrosine residues. These signaling proteins are involved in the pathways by which activated receptor Tyrosine kinases transduce signals into the nucleaus to regulate gene expression and so influence proliferation and differentiation of cells. To the date, Ras protein- involved pathway is the most clearly understood one. Ras protein is the product of Ras gene, consisting of 190 amino acid residues and having a molecular weight of 21KD. Ras protein is anchored on intracelluar surface of plasma membrane by covalently binding to isopentenyl. Ras is not a kinase. Since Ras protein is activated when binding GTP and repressed when binding GDP, functionally it could be regarded as a kind of molecular switching. After the activation of Ras, Raf proteins locating in cytoplasm move to near the inner surface of plasma membrane and will be activated by Ras after their binding. Raf is a kind of Ser/Thr protein kinases which can phosporylate Ser/Thr residues of proteins. Downstream signal transduction process after the activation of Raf involves Many Ser/Thr residue phosphorylation cascade reactions, among which mitogen activated protein kinase(MAPK) is an especially important one. Activated MAPK will enter the nucleus to regulate transcription of some genes including Jun, Elk-1 and others.

SHC genes encode a signal transducer protein family containing Src homologous two and three (SH2 and SH3) functional domains. SHC proteins are adaptors which have no catalysis activity but can couple activated receptor Tyrosine kinases to other proteins without SH2 and SH3 domains. Mutation of SHC proteins may cause the block of receptor Tyrosine kinase pathway.

Structure analysis reveals that members of SHC family all contain a C-terminal SH2 domain, and a Proline and Glycine rich region close together. Please find its detailed structure in corresponding reference. Cell 1992 Jul 10; 70(1):93-104

There are two highly conserved but not catalytically active domains SH2 and SH3 in SHC proteins. SH2 domain can recognize phosphorylated Tyrosine residues and help proteins containing SH2 bind to activated receptor Tyrosine kinases or other signaling proteins owning transiently phosphorylated Tyrosine residues. SH3 can bind to Proline rich region in target proteins to transduce signals.

SHC proteins can be quickly phosphorylated by some growth factor receptors having Tyrosine kinase activity. They have no catalytic activity but can couple activated receptor Tyrosine kinases to other proteins without SH2 and SH3 domains. SHC's function also include facilitation of Ras activation which is important to Ras pathway. And indirectly, SHC proteins also play an important role in the MAPK signal transduction pathway.

SHC proteins have many biological functions such as to regulate cell proliferation, control signal transduction and in vivo expression of kinds of proteins whose abnormal expression will lead to abnormal proliferation of tissue cells, abnormal expression of other proteins and occurrence of corresponding diseases such as malignant tumors, cancers, developmental disorders, and immune system diseases.

According to amino acid homologous comparison analysis, the polypeptide of the present invention is determined as a novel human SHC protein 43 HSHC43 whose homologous protein is the early found human SHC protein (protein SN. X68148).

As described above, human SHC protein 43 plays an essential role in the regulation of important biological functions such as cell division and embryogenesis, and it's believed that many of proteins are involved in these regulations. So the determination of those related human SHC proteins 43, especially of their amino acid sequences is always desired in this filed. The isolation of this novel human SHC protein 43 builds the basis for research of the protein function under normal and clinical conditions, disease diagnosis and drug development. So the isolation of its cDNA is very important.

Disclosure of Invention

One objective of the invention is to provide an isolated novel polypeptide, i.e., a human SHC protein 43, and fragments, analogues and derivatives thereof.

Another objective of the invention is to provide a polynucleotide encoding said polypeptide.

Another objective of the invention is to provide a recombinant vector containing a polynucleotide encoding a human SHC protein 43.

Another objective of the invention is to provide a genetically engineered host cell containing a polynucleotide encoding a human SHC protein 43.

Another objective of the invention is to provide a method for producing a human SHC protein 43.

Another objective of the invention is to provide an antibody against a human SHC protein 43 of the invention.

Another objective of the invention is to provide mimetics, antagonists, agonists, and inhibitors for the polypeptide of the human SHC protein 43.

Another objective of the invention is to provide a method for the diagnosis and treatment of the diseases associated with an abnormality of human SHC protein 43.

The present invention relates to an isolated polypeptide, which is originated from human, and comprises a polypeptide having the amino acid sequence of SEQ ID NO: 2, or its conservative variants, or its active fragments, or its active derivatives and its analogues. Preferably, the polypeptide has the amino acid sequence of SEQ ID NO: 2.

The present invention also relates to an isolated polynucleotide, comprising a nucleotide sequence or its variant selected from the group consisting of (a) the polynucleotide encodeing a polypeptide comprising the amino acid sequence of SEQ ID NO: 2; and (b) a polynucleotide complementary to the polynucleotide (a);(c) a polynucleotide that shares at least 70% homology to the polynucleotide (a) or (b). Preferably, said nucleotide sequence is selected from the group consisting of (a) the sequence of position 996-2159 in SEQ ID NO: 1; and (b) the sequence of position 1-2719 in SEQ ID NO: 1.

The invention also includes: a vector containing a polynucleotide of said invention, especially an expression vector; a host cell genetically engineered with the vector via transformation, transduction or transfection; a method for the production of said inventive polypeptide through the process of host cell cultivation and expression product harvest.

The invention also relates to an antibody which specifically binds to the inventive polypeptide.

The invention also relates to a method for selecting compounds which could simulate, activate, antagonize, or inhibit the activity of the inventive polypeptide and the compounds obtained by the method.

The invention also relates to a method for *in vitro* diagnosis method of the diseases or disease susceptibility related with the abnormal expression of the inventive polypeptide. The method involves the detection of mutation in the polypeptide or its encoding polynucleotide sequence, or the determination of its quantity and/or biological activity in biological samples.

The invention also relates to pharmaceutical compositions which comprises the inventive polypeptide, its analogues, mimetics, agonists, antagonists, inhibitors, and a pharmaceutically acceptable carrier.

The invention also relates to applications of the inventive polypeptide and/or its polynucleotide for drug development to treat cancers, developmental diseases, immune diseases, or other diseases caused by abnormal expression of the inventive polypeptide.

Other aspects of the invention are apparent to the skilled in the art in view of the disclosure set forth hereinbelow.

The terms used in this specification and claims have the following meanings, unless otherwise noted.

"Nucleotide sequence" refers to oligonucleotide, nucleotide, or polynucleotide and parts of polynucleotide. It also refers to genomic or synthetic DNA or RNA, which could be single stranded or double stranded, and could represent the sense strand or the antisense strand. Similarly, the term "amino acid sequence" refers to oligopeptide, peptide, polypeptide, or protein sequence and parts of proteins. When the "amino acid sequence" in the invention is related to the sequence of a natural protein, the amino acid sequence of said "peptide" or "protein" will not be limited to be identical to the sequence of that natural protein.

"Variant" of a protein or polynucleotide refers to the amino acid sequence or nucleotide sequence, respectively with one or more amino acids or one or more nucleotides changed. Such changes include deletion, insertion, and/or substitution of amino acids in the animo acid sequence, or of nucleotides in the polynucleotide sequence. These changes could be conservative and the substituted amino acid has similar structural or chemical characteristics as the original one, such as the substitution of Ile with Leu. Changes also could be not conservative, such as the substitution of Ala with Trp.

"Deletion" refers to the deletion of one or several amino acids in the amino acid sequence, or of one or several nucleotides in the nucleotide sequence.

"Insertion" or "addition" refers to the addition of one or several amino acids in the amino acid sequence, or of one or several nucleotides in the nucleotide sequence, comparing to the natural molecule. "Substitution" refers to the change of one or several amino acids, or of one or several nucleotides, into different ones without changing number of the residues.

"Biological activity" refers to structural, regulatory or biochemical characteristics of a natural molecule. Similarly, the term "immungenecity" refers to the ability of natural, recombinant, or synthetic proteins to inducing a specific immunologic reaction, or of binding specific antibody in appropriate kind of animal or cell.

"Agonist" refers to molecules which regulate, but generally enhance the activity of the inventive polypeptide by binding and changing it. Agonists include proteins, nucleotides, carbohydrates or any other molecules which could bind the inventive polypeptide.

"Antagonist" or "inhibitor" refers to molecules which inhibit or downregulate the biological activity or immunogenecity the inventive polypeptide via binding to it. Antagonists or inhibitors include proteins, nucleotides, carbohydrates or any other molecules which bind to the inventive polypeptide.

"Regulation" refers to changes in function of the inventive polypeptide, including up-regulation or down-regulation of the protein activity, changes in binding specifity, changes of any other biological characteristics, functional or immune characteristics.

"Substantially pure" refers to the condition of substantially free of other naturally related proteins, lipids, saccharides, or other substances. One of ordinary skill in the art can purify the inventive polypeptide by standard protein purification techniques. Substantially pure polypeptide of the invention produces a single main band in a denaturing polyacrylamide gel. The purity of a polypeptide may also be analyzed by amino acid sequence analysis.

Complementary" or complementation refers to the binding of polynucleotides by base pairing under the condition of approximate ion conditions and temperature. For instance, the sequence C-T-G-A could bind its complementary sequence G-A-C-T." The complementation between two single strand molecules could be partial or complete. Homology or sequence similarity between two single strands obviously influences the efficiency and strength of the formed hybrid.

"Homology" refers to the complementary degree, which may be partially or completely homologous. "Partial homology " refers to a sequence being partially complementary to a target nucleotide. The sequence could at least partially inhibit the hybridization between a completely complementary sequence and the target nucleotide. Inhibition of hybridization could be assayed by hybridization (Southern blot or Northern blot) under less stringent conditions. Substantially complementary sequence or hybrid probe could compete with the completely complementary sequence and inhibit its hybridization with the target sequence under less stringent conditions. This doesn't mean that nonspecific binding is allowed under a less stringent condition, because specific or selective reaction is still required.

"Sequence Identity" refers to the percentage of sequence identity or similarity when two or several amino acid or nucleotide sequences are compared. Sequence identity may be determined by computer programs such as MEGALIGN (Lasergene Software Package, DNASTAR, Inc., Madison Wis.). MEGALIGN can compare two or several sequences using different methodologies such as the Cluster method (Higgins, D. G. and P.M. Sharp (1988) Gene 73: 237-244). Cluster method examines the distance between all pairs and arrange the sequences into clusters. Then the clusters are partitioned by pair or group. The sequence identity between two amino acid sequences such as sequence A and B can be calculated by the following equation:

$$\frac{\text{Number of paired identical residues between sequences A and B}}{\text{Residue number of sequence A - number of gap residues in sequence A}} \times 100$$
$$\text{- number of gap residue in sequence B}$$

Sequence identity between nucleotide sequences can also be determined by Cluster method or other well-known methods in the art such as the Jotun Hein method (Hein J., (1990) Methods in Emzymology 183:625-645)

"Similarity" refers to the degree of identity or conservative substitution degree of amino acid residues in corresponding sites of the amino acid sequences when compared to each other. Amino acids for conservative substitution are: negative charged amino acids including Asp and Glu; positive charged amino acids including Leu, Ile and Val; Gly and Ala; Asn and Gln; Ser and Thr; Phe and Tyr.

"Antisense" refers to the nucleotide sequences complementary to a specific DNA or RNA sequence. "Antisese strand" refers to the nucleotide strand complementary to the "sense strand."

"Derivative" refers to the inventive polypeptide or the chemically modified nucleotide encoding it. This kind of modified chemical can be derived from replacement of the hydrogen atom with Alkyl, Acyl, or Amino. The nucleotide derivative can encode peptide retaining the major biological characteristics of the natural molecule.

"Antibody" refers to the intact antibody or its fragments such as Fa, F(ab')2 and Fv, and it can specifically bind to antigenic epitopes of the inventive polypeptide.

"Humanized antibody" refers to an antibody which has its amino acid sequence in non-antigen binding region replaced to mimic human antibody and still retain the original binding activity.

The term "isolated" refers to the removal of a material out of its original environment (for instance, if it's naturally produced, original environment refers to its natural environment). For example, a naturally produced polynucleotide or a polypeptide in its original host organism means it has not been "isolated," while the separation of the polynucleotide or a polypeptide from its coexisting materials in natural system means it was "isolated." This polynucleotide may be a part of a vector, or a part of a compound. Since the vector or compound is not part of its natural environment, the polynucleotide or peptide is still "isolated."

As used herein, the term "isolated" refers to a substance which has been isolated from the original environment. For naturally occurring substance, the original environment is the natural environment. For example, the polynucleotide and polypeptide in a naturally occurring state in the viable cells are not isolated or purified. However, if the same polynucleotide and polypeptide have been isolated from other components naturally accompanying them, they are isolated or purified.

As used herein, "isolated human SHC protein 43," means that human SHC protein 43 does not essentially contain other proteins, lipids, carbohydrate or any other substances associated therewith in nature. The skilled in the art can purify human SHC protein 43, by standard protein purification techniques. The purified polypeptide forms a single main band on a non-reducing PAGE gel. The purity of human SHC protein 43 can also be analyzed by amino acid sequence analysis.

The invention provides a novel polypeptide - human SHC protein 43, which comprises the amino acid sequence shown in SEQ ID NO: 2. The polypeptide of the invention may be a recombinant polypeptide, natural polypeptide, or synthetic polypeptide, preferably a recombinant polypeptide. The polypeptide of the invention may be a purified natural product or a chemically synthetic product. Alternatively, it may be produced from prokaryotic or eukaryotic hosts, such as bacterial, yeast, higher plant, insect, and mammal cells, using recombinant techniques. Depending on the host used in the protocol of recombinant production, the polypeptide of the invention may be glycosylated or non-glycosylated. The polypeptide of the invention may or may not comprise the starting Met residue.

The invention further comprises fragments, derivatives and analogues of human SHC protein 43. As used in the invention, the terms "fragment," "derivative" and "analogue" mean the polypeptide that essentially retains the same biological functions or activity of human SHC protein 43 of the invention. The fragment, derivative or analogue of the polypeptide of the invention may be (i) one in which one or more of the amino acid residues are substituted with a conserved or non-conserved amino acid residue (preferably a conserved amino acid residue) and such substituted amino acid residue may or may not be one encoded by the genetic code; or (ii) one in which one or more of the amino acid residues are substituted with other residues, including a substituent group; or (iii) one in which the mature polypeptide is fused with another compound, such as a compound to increase the half-life of the polypeptide (for example, polyethylene glycol); or (iv) one in which additional amino acids are fused to the mature polypeptide, such as a leader or secretory sequence or a sequence which is employed for purification of the mature polypeptide or a proprotein sequence. Such fragments, derivatives and analogs are deemed to be within the scope of the skilled in the art from the teachings herein.

The invention provides an isolated nucleic acid or polynucleotide which comprises the polynucleotide encoding an amino acid sequence of SEQ ID NO: 2. The polynucleotide sequence of the invention includes the nucleotide sequence of SEQ ID NO: 1. The polynucleotide of the invention was identified in a human embryonic brain cDNA library. Preferably, it comprises a full-length polynucleotide sequence of 2719 bp, whose ORF (996-2159) encodes 387 amino acids. Based on amino acid homology comparison, it is found that the encoded polypeptide is 54% homologous to the early found human SHC protein. This novel human SHC protein 43 has similar structures and biological functions to those of the early found human SHC protein.

The polynucleotide according to the invention may be in the forms of DNA or RNA. The forms of DNA include cDNA, genomic DNA, and synthetic DNA, etc., in single stranded or double stranded form. DNA may be an encoding strand or a non-encoding strand. The coding sequence for mature polypeptide may be identical to the coding sequence shown in SEQ ID NO: 1, or is a degenerate sequence. As used herein, the term "degenerate sequence" means a sequence which encodes a protein or peptide comprising a sequence of SEQ ID NO: 2 and which has a nucleotide sequence different from the sequence of coding region in SEQ ID NO: 1.

The polynucleotide encoding the mature polypeptide of SEQ ID NO: 2 includes those encoding only the mature polypeptide, those encoding mature polypeptide plus various additional coding sequence, the coding sequence for mature polypeptide (and optional additional encoding sequence) plus the non-coding sequence.

The term "polynucleotide encoding the polypeptide " includes polynucleotides encoding said polypeptide and polynucleotides comprising additional coding and/or non-coding sequences.

The invention further relates to variants of the above polynucleotides which encode a polypeptide having the same amino acid sequence of invention, or a fragment, analogue and derivative of said polypeptide. The variant of the polynucleotide may be a naturally occurring allelic variant or a non-naturally occurring variant. Such nucleotide variants include substitution, deletion, and insertion variants. As known in the art, an allelic variant may have a substitution, deletion, and insertion of one or more nucleotides without substantially changing the functions of the encoded polypeptide.

The present invention further relates to polynucleotides, which hybridize to the hereinabove-described sequences, that is, there is at least 50% and preferably at least 70% identity between the sequences. The present invention particularly relates to polynucleotides, which hybridize to the polynucleotides of the invention under stringent conditions. As herein used, the term "stringent conditions" means the following conditions: (1) hybridization and washing under low ionic strength and high temperature, such as 0.2xSSC, 0.1% SDS, 60°C; or (2) hybridization after adding denaturants, such as 50% (v/v) formamide, 0.1% bovine serum/0.1% Ficoll, 42°C; or (3) hybridization only when the homology of two sequences at least 95%, preferably 97%. Further, the polynucleotides which hybridize to the hereinabove described polynucleotides encode a polypeptide which retains the same biological function and activity as the mature polypeptide of SEQ ID NO: 2

The invention also relates to nucleic acid fragments hybridized with the hereinabove sequence. As used in the present invention, the length of the "nucleic acid fragment " is at least more than 10 bp, preferably at least 20-30 bp, more preferably at least 50-60 bp, and most preferably at least 100 bp. The nucleic acid fragment can be used in amplification techniques of nucleic acid, such as PCR, so as to determine and/or isolate the polynucleotide encoding human SHC protein 43.

The polypeptide and polynucleotide of the invention are preferably in the isolated form, preferably purified to be homogenous.

According to the invention, the specific nucleic acid sequence encoding human SHC protein 43 can be obtained in various ways. For example, the polynucleotide is isolated by hybridization techniques well-known in the art, which include, but are not limited to 1) the hybridization between a probe and genomic or cDNA library so as to select a homologous polynucleotide sequence, and 2) antibody screening of expression library so as to obtain polynucleotide fragments encoding polypeptides having common structural features.

According to the invention, DNA fragment sequences may further be obtained by the following methods: 1) isolating double-stranded DNA sequence from genomic DNA; and 2) chemical synthesis of DNA sequence so as to obtain the double-stranded DNA.

Among the above methods, the isolation of genomic DNA is least frequently used. A commonly used method is the direct chemical synthesis of DNA sequence. A more frequently used method is the isolation of cDNA sequence. Standard methods for isolating the cDNA of interest is to isolate mRNA from donor cells that highly express said gene followed by reverse transcription of mRNA to form plasmid or phage cDNA library. There are many established techniques for extracting mRNA and the kits are commercially available (e.g. Qiagene). Conventional method can be used to construct cDNA library (Sambrook, et al., Molecular Cloning, A Laboratory Manual, Cold Spring Harbor Laboratory. New York, 1989). The cDNA libraries are also commercially available. For example, Clontech Ltd. has various cDNA libraries. When PCR is further used, even an extremely small amount of expression products can be cloned.

Numerous well-known methods can be used for screening for the polynucleotide of the invention from cDNA library. These methods include, but are not limited to, (1) DNA-DNA or DNA-RNA hybridization; (2) the appearance or loss of the function of the marker-gene; (3) the determination of the level of human SHC protein 43 transcripts; (4) the determination of protein product of gene expression by immunology methods or the biological activity assays. The above methods can be used alone or in combination.

In method (1), the probe used in the hybridization could be homologous to any portion of polynucleotide of invention. The length of probe is typically at least 10 nucleocides, preferably at least 30 nucleocides, more preferably at least 50 nucleocides, and most preferably at least 100 nucleotides. Furthermore, the length of the probe is usually less than 2000 nucleotides, preferably less than 1000 nucleotides. The probe usually is the DNA sequence chemically synthesized on the basis of the sequence information. Of course, the gene of the invention itself or its fragment can be used as a probe. The labels for DNA probe include, e.g., radioactive isotopes, fluoresceins or enzymes such as alkaline phosphatase.

In method (4), the detection of the protein products expressed by human SHC protein 43 gene can be carried out by immunology methods, such as Western blotting, radioimmunoassay, and ELISA.

The method of amplification of DNA/RNA by PCR (Saiki, et al. Science 1985; 230:1350-1354) is preferably used to obtain the polynucleotide of the invention. Especially when it is difficult to obtain the full-length cDNA, the method of RACE (RACE- cDNA terminate rapid amplification) is preferably used. The primers used in PCR can be selected according to the polynucleotide sequence information of the invention disclosed herein, and can be synthesized by conventional methods. The amplified DNA/RNA fragments can be isolated and purified by conventional methods such as gel electrophoresis.

Sequencing of polynucleotide sequence of the gene of the invention or its various DNA fragments can be carried out by the conventional dideoxy sequencing method (Sanger et al. PNAS, 1977, 74: 5463-5467). Sequencing of polynucleotide sequence can also be carried out using the commercially available sequencing kits. In order to obtain the full-length cDNA sequence, it is necessary to repeat the sequencing process. Sometimes, it is needed to sequence the cDNA of several clones to obtain the full-length cDNA sequence.

The invention further relates to a vector comprising the polynucleotide of the invention, a genetically engineered host cell transformed with the vector of the invention or directly with the sequence encoding human SHC protein 43, and a method for producing the polypeptide of the invention by recombinant techniques.

In the present invention, the polynucleotide sequences encoding human SHC protein 43 may be inserted into a vector to form a recombinant vector containing the polynucleotide of the invention. The term "vector" refers to a bacterial plasmid, bacteriophage, yeast plasmid, plant virus or mammalian virus such as adenovirus, retrovirus or any other vehicle known in the art. Vectors suitable for use in the present invention include, but are not limited to the T7-based expression vector for expression in bacteria (Rosenberg, et al., Gene, 56:125, 1987), the pMSXND expression vector for expression in mammalian cells (Lee and Nathans, J Biol. Chem., 263:3521, 1988) and baculovirus-derived vectors for expression in insect cells. Any plasmid or vector can be used to construct the recombinant expression vector as long as it can replicate and is stable in the host. One important feature of an expression vector is that the expression vector typically contains an origin of replication, a promoter, a marker gene as well as translation regulatory components.

Methods known in the art can be used to construct an expression vector containing the DNA sequence of human SHC protein 43 and appropriate transcription/translation regulatory components. These methods include in vitro recombinant DNA technique, DNA synthesis technique, in vivo recombinant technique and so on (Sambroook, et al. Molecular Cloning, a Laboratory Manual, cold Spring Harbor Laboratory. New York, 1989). The DNA sequence is operatively linked to a proper promoter in an expression vector to direct the synthesis of mRNA. Exemplary promoters are lac or trp promoter of E.coli; PL promoter of λ phage; eukaryotic promoters including CMV immediate early promoter, HSV thymidine kinase promoter, early and late SV40 promoter, LTRs of retrovirus, and other known promoters which control gene expression in the prokaryotic cells, eukaryotic cells or viruses. The expression vector may further comprise a ribosome binding site for initiating translation, transcription terminator and the like. Transcription in higher eukaryotes is increased by inserting an enhancer sequence into the vector. Enhancers are cis-acting elements of DNA, usually about from 10 to 300 bp in length that act on a promoter to increase gene transcription level. Examples include the SV40 enhancer on the late side of the replication origin 100 to 270 bp, the polyoma enhancer on the late side of the replication origin, and adenovirus enhancers.

Further, the expression vector preferably comprises one or more selective marker genes to provide a phenotype for the selection of the transformed host cells, e.g., the dehydrofolate reductase, neomycin resistance gene and GFP (green flurencent protein) for eukaryotic cells, as well as tetracycline or ampicillin resistance gene for *E. coli.*

An ordinarily skilled in the art know clearly how to select appropriate vectors, transcriptional regulatory elements, e.g., promoters, enhancers, and selective marker genes.

According to the invention, polynucleotide encoding human SHC protein 43 or recombinant vector containing said polynucleotide can be transformed or transfected into host cells to construct genetically engineered host cells containing said polynucleotide or said recombinant vector. The term "host cell" means prokaryote, such as bacteria; or primary eukaryote, such as yeast; or higher eukaryotic, such as mammalian cells. Representative examples are bacterial cells, such as E. coli, Streptomyces, Salmonella typhimurium; fungal cells, such as yeast; plant cells; insect cells such as Drosophila S2 or Sf9; animal cells such as CHO, COS or Bowes melanoma.

Transformation of a host cell with the DNA sequence of invention or a recombinant vector containing said DNA sequence may be carried out by conventional techniques as are well known to those skilled in the art. When the host is prokaryotic, such as E. coli, competent cells, which are capable of DNA uptake, can be prepared from cells harvested after exponential growth phase and subsequently treated by the CaCl2 method using procedures well known in the art. Alternatively, MgCl2 can be used. Transformation can also be carried out by electroporation, if desired. When the host is an eukaryote, transfection methods as well as calcium phosphate precipitation may be used. Conventional mechanical procedures such as micro-injection, electroporation, or liposome-mediated transfection may also be used.

The recombinant human SHC protein 43 can be expressed or produced by the conventional recombinant DNA technology (Science, 1984; 224:1431), using the polynucleotide sequence of the invention. The steps generally include:

(1) transfecting or transforming the appropriate host cells with the polynucleotide (or variant) encoding human SHC protein 43 of the invention or the recombinant expression vector containing said polynucleotide;

(2) culturing the host cells in an appropriate medium; and

(3) isolating or purifying the protein from the medium or cells.

In Step (2) above, depending on the host cells used, the medium for cultivation can be selected from various conventional mediums. The host cells are cultured under a condition suitable for its growth until the host cells grow to an appropriate cell density. Then, the selected promoter is induced by appropriate means (e.g., temperature shift or chemical induction) and cells are cultured for an additional period.

In Step (3), the recombinant polypeptide may be included in the cells, or expressed on the cell membrane, or secreted out of the cell. If desired, physical, chemical and other properties can be utilized in various isolation methods to isolate and purify the recombinant protein. These methods are well-known to those skilled in the art and include, but are not limited to conventional renaturation treatment, treatment by a protein precipitant (such as salt precipitation), centrifugation, cell lysis by osmosis, sonication, supercentrifugation, molecular sieve chromatography or gel chromatography, adsorption chromatography, ion exchange chromatography, HPLC, and any other liquid chromatography, and a combination thereof.

Brief Description of the Drawings

The following drawings are provided to illustrate the embodiment of the invention, not to limit the scope of invention defined by the claims.

Fig. 1 shows an alignment comparison of amino acid sequences of human SHC protein 43 of the invention and human SHC protein. The upper sequence is human SHC protein 43, and the lower sequence is human SHC protein. The identical and similar amino acids are indicated by a one-letter code of amino acid and "+" respectively.

Fig. 2 shows the SDS-PAGE of the isolated human SHC protein 43, which has a molecular weight of 43kDa. The isolated protein band is marked with an arrow.

Examples

The invention is further illustrated by the following examples. It is appreciated that these examples are only intended to illustrate the invention, not to limit the scope of the invention. For the experimental methods in the following examples, they are performed under routine conditions, e.g., those described by Sambrook. et al., in Molecule Clone: A Laboratory Manual, New York: Cold Spring Harbor Laboratory Press, 1989, or as instructed by the manufacturers, unless otherwise specified.

**Example 1 Cloning of Human SHC protein 43 Gene**

Total RNA from a human embryonic brain was extracted by the one-step method with guanidinium isocyanate/ phenol/chloroform. The poly(A) mRNA was isolated from the total RNA with Quik mRNA Isolation Kit (Qiegene). cDNA was prepared by reverse transcription with 2 µg poly(A) mRNA. The cDNA fragments were inserted into the polyclonal site of pBSK(+) vector (Clontech) using Smart cDNA cloning kit (Clontech) and then transformed into DH5α to form the cDNA library. The 5'- and 3'-ends of all clones were sequenced with Dye terminate cycle reaction sequencing kit (Perkin-Elmer) and ABI 377 Automatic Sequencer (Perkin-Elmer). The sequenced cDNA were compared with the public database of DNA sequences (Genebank) and the DNA sequence of one clone 1002e04 was found to be a novel DNA sequence. The inserted cDNA sequence of clone 1002e04 was dual-directionally sequenced with a serial of synthesized primers. It was indicated that the full length cDNA contained in clone 1002e04 was 2719bp (SEQ ID NO: 1) with a 1164bp ORF located in positions 996-2159 which encoded a novel protein (SEQ ID NO: 2). This clone was named pBS-1002e04 and the encoded protein was named human SHC protein 43.

**Example 2 Homology Search of cDNA Clone**

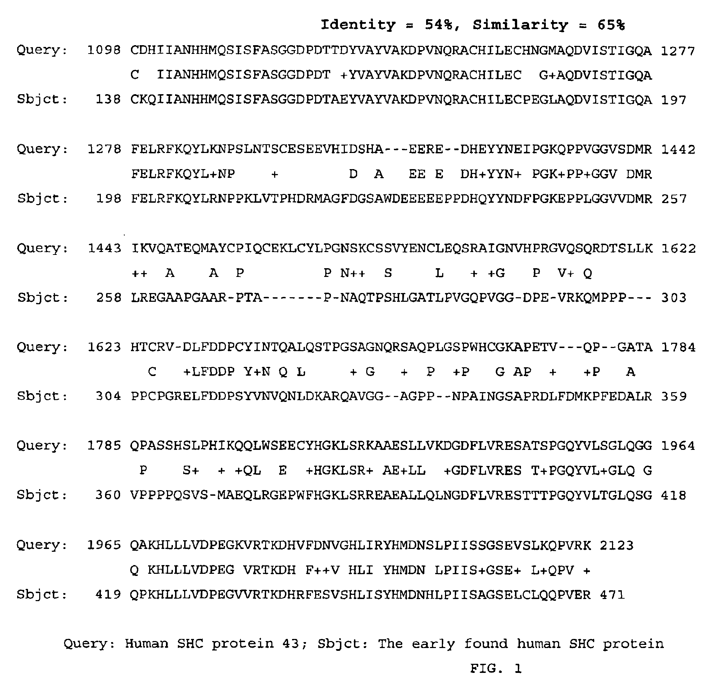

The homology research of the DNA sequence and its protein sequence of human SHC protein 43 of the invention were performed by Blast (Basic Local Alignment Search Tool) (Altschul, SF et al. J.Mol.Biol. 1990; 215: 403-10) in databases such as Genbank, Swissport, etc. The most homologous gene to human SHC protein 43 of human SHC protein. The Genbank accession number of its encoded protein is X68148. The alignment result of the protein was shown in Fig. 1. The two proteins are highly homologous with an identity of 54% and a similarity of 65%.

**Example 3 Cloning Human SHC protein 43 Gene by RT-PCR**

The template was total RNA extracted from a human embryonic brain. The reverse transcription was carried out with oligo-dT primer to produce cDNAs. After cDNA purified with Qiagen Kit, PCR was carried out with the following primers:

Primer1:   5'- GTACTTTTTTTTTTTTAGTTATAGT -3'   (SEQ ID NO:3)

Primer2:   5'- CGAATTCAATGTCATATTTATTTT -3'   (SEQ ID NO:4)

Primer 1 is the forward sequence started from position 1 of 5' end of SEQ ID NO: 1.
Primer 2 is the reverse sequence of the 3' end of SEQ ID NO: 1.
The amplification condition was a 50 μl reaction system containing 50mmol/L KCl, 10mmol/L Tris-Cl (pH8.5), 1.5mmol/L $MgCl_2$, 200umol/L dNTP, 10pmol of each primer, 1U Taq DNA polymerase(Clontech). The reaction was performed on a PE 9600 DNA amplifier with the following parameters: 94°C 30 sec, 55°C 30sec, and 72°C 2min for 25 cycles. β-actin was used as a positive control, and a blank template, as a negative control in RT-PCR. The amplified products were purified with a QIAGEN kit, and linked with a pCR vector (Invitrogen) using a TA Cloning Kit. DNA sequencing results show that the DNA sequence of PCR products was identical to nucleotides 1-2719bp of SEQ ID NO: 1.

**Example 4 Northern Blotting of Expression of Human SHC protein 43 Gene**

Total RNA was extracted by one-step method (Anal. Biochem 1987, 162, 156-159) with guanidinium isocyanate-phenol-chloroform. That is, homogenate the organize using 4M guanidinium isocyanate-25mM sodium citrate and 0.2M sodium acetate, add 1 volume phenol and 1/5 volume chloroform-isoamyl alcohol(49:1), centrifuge after mixing. Take out the water phase, add 0.8 volume isopropyl alcohol, then centrifuge the mixture. Wash the RNA precipitation using 70% ethanol, then dry, then dissolve it in the water. 20· g RNA was electrophoresed on the 1.2% agarose gel containing 20mM 3-(N-morpholino) propane sulfonic acid(pH 7.0)-5mM sodium acetate-imM EDTA- 2.2M formaldehyde. Then transfer it to a nitrocellulose filter. Prepare the $^{32}$P-labelled DNA probe with · -$^{32}$P dATP by random primer method. The used DNA probe is the coding sequence (996bp-2159bp) of human SHC protein 43 amplified by PCR indicated in figure 1. The nitrocellulose filter with the transferred RNA was hybridized with the $^{32}$P-labelled DNA probe ($2\times10^6$cpm/ml) overnight in a buffer containing 50% formamide-25mM $KH_2PO_4$(Ph7.4)-5xDenhardt's solution and 200· g /ml salmine. Then wash the filter in the 1$\bar{7}$SSC-0.1% SDS, at 55°C, for 30 min. Then analyze and quantitative determinate using Phosphor Imager.

**Example 5 *In vitro* Expression, Isolation and Purification of Recombinant Human SHC protein 43**

A pair of primers for specific amplification was designed based on SEQ ID NO: 1 and the encoding region in Fig.1, the sequences are as follows:

Primer3:  5'-  CATGCTAGCATGCTTCCTGCCCTCGAACATTGGA  -3' ·  Seq  ID  No:5·

Primer4: 5'- CCCGAGCTCTCATTTGTTGGAATGCAAAAGTGCT -3' ·  Seq ID No:6

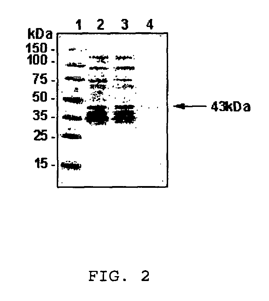

These two primers contain a NdeI and SacI cleavage site on the 5' end respectively. Within the sites are the coding sequences of the 5' and 3' end of the desired gene. NdeI and SacI cleavage sites were corresponding to the selective cleavage sites on the expression vector pET-28b(+) (Novagen, Cat. No. 69865.3). PCR amplification was performed with the plasmid pBS-1002e04 containing the full-length target gene as a template. The PCR reaction was subject to a 50μl system containing 10pg pBS-1002e04 plasmid, 10pmol of Primer-3 and 10pmol of Primer-4, 1μl of Advantage polymerase Mix (Clontech). The parameters of PCR were 94 °C 20 sec, 60 °C 30 sec, and 68 °C 2 min for 25 cycles. After digesting the amplification products and the plasmid pET-28(+) by NdeI and SacI, the large fragments were recovered and ligated with T4 ligase. The ligated product was transformed into *E.coli* DH5α with the calcium chloride method. After cultured overnight on a LB plate containing a final concentration of 30μg/ml kanamycin, positive clones were selected out using colony PCR and then sequenced. The positive clone (pET-1002e04) with the correct sequence was selected out and the recombinant plasmid thereof was transformed into BL21(DE3)plySs (Novagen) using the calcium chloride method. In a LB liquid medium containing a final concentration of 30μg/ml of kanamycin, the host bacteria BL21(pET-1002e04) were cultured at 37°C to the exponential growth phase, then IPTG were added with the final concentration of 1 mmol/L, the cells were cultured for another 5 hours, and then centrifuged to harvest the bacteria. After the bacteria were sonicated, the supernatant was collected by centrifugation. Then the purified desired protein - human SHC protein 43 was obtained by a His.Bind Quick Cartridge (Novagen) affinity column with binding 6His-Tag. SDS-PAGE showed a single band at 43 kDa (Fig.2). The band was transferred onto the PVDF membrane and the N terminal amino acid was sequenced by Edams Hydrolysis, which shows that the first 15 amino acids on N-terminus were identical to those in SEQ ID NO: 2.

**Example 6 Preparation of Antibody Against Human SHC protein 43**

The following specific human SHC protein 43 polypeptide was synthesized by a polypeptide synthesizer (PE-ABI): NH2-Met-Leu-Pro-Ala-Leu-Glu-His-Trp-Ile-Pro-Lys-Phe-Phe-Ser-Phe-COOH (SEQ ID NO:7). The polypeptide was conjugated with hemocyanin and bovine serum albumin (BSA) respectively to form two composites (See Avrameas et al., Immunochemistry, 1969, 6:43). 4 mg of hemocyanin-polypeptide composite was used to immunize rabbit together with Freund's complete adjuvant. The rabbit was re-immunized with the hemocyanin-polypeptide composite and Freund's incomplete adjuvent 15 days later. The titer of antibody in the rabbit sera was determined with a titration plate coated with 15μg/ml BSA-polypeptide composite by ELISA. The total IgG was isolated from the sera of an antibody positive rabbit with protein A-Sepharose. The polypeptide was bound to Sepharose 4B column activated by cyanogen bromide. The antibodies against the polypeptide were isolated from the total IgG by affinity chromatography. The immunoprecipitation approved that the purified antibodies could specifically bind to human SHC protein 43.

**Example 7 Application of the Polynucleotide Fragments of the Invention as Hybridization Probes**

Oligonucleotides probes selected from the polynucleotide of the invention have many applications. The probe could be used to determine the existence of polynucleotide of the invention or its homologous polynucleotide sequences by hybridization with genomic, or cDNA library of normal or clinical tissues from varied sources. The probes could be further used to determine whether polynucleotide of the invention or its homologous polynucleotide sequences are abnormally expressed in cells from normal or clinical tissues.

The purpose of the following example is to select suitable oligonucletide fragments from SEQ ID NO:1 as hybird probes to apply in membrane hybridization to determine whether there is polynucleotide of the invention or its homologous polynucleotide sequences in sample tissues. Membrane hybridization methods include dot blot, Southern blot, Northern blot, and replica hybridization. All methods follow nearly the same steps after the polynucleotide samples are immobilized on membranes. These steps are: membranes with immobilized samples are prehybridized in hybridization buffer not containing probes to block nonspecific binding sites of the membranes. Then the prehybridization buffer is replaced by hybridization buffer containing labeled probes and incubation is carried out at the appropriate temperature so probes hybridize with the target nucleotides. Free probes are washed off by a series of washing steps after the hybridization step. A high-stringency washing condition (relatively low salt concentration and high temperature) is applied in the example to reduce background and retain highly specific signals. Two types of probes are selected for the example: the first type of probes are oligonucleotides identical or annealed to SEQ ID NO:1; the second type probes are oligonucleotides partially identical or partially annealed to SEQ ID NO:1. Dot blot method is applied in the example for immobilization of the samples on membrane. The strongest specific signal produced by hybridization between first type probes and samples is obtained after relatively strict membrane washing steps.

<u>Selection of Probes</u>

The principles below should be followed for the selection of oligonucleotide fragments from SEQ ID NO:1 as hybrid probes:

1. The optimal length of probes should be between eighteen and fifty nucleotides.

2. GC amount should be between 30% and 70%, since nonspecific hybridization increases when GC amount is more than 70%.

3. There should be no complementary regions within the probes themselves.

4. Probes meeting the requirements above could be initially selected for further computer-aided sequence analysis, which includes homology comparison between the initially selected probes and its source sequence region (SEQ ID NO: 1), other known genomic sequences and their complements. Generally, the initial selected probes should not be used when they share fifteen identical continuous base pairs, or 85% homology with non-target region.

5. Whether the initially selected probes should be chosen for final application depends upon further experimental confirmation.

The following two probes could be selected and synthesized after the analysis above:

Probe One belongs to the first type, which is completely identical or annealed to the gene fragments of SEQ ID NO: 1 (41 nucleotides):

5'-CCAAGTTCTTCAGTTTCAGAACTCGGACTGGCTCTCCTCTC-3' · SEQ ID NO: 8·

Probe Two belongs to the second type which is a substituted or mutant sequence of a fragment of SEQ ID NO: 1 (41 nucleotides):

5'-CCAAATCGTTCAGTTTCAGATCGCGGAATCGCTCTATCGTC-3' · SEQ ID NO: 9·

Any other frequently used reagents unlisted but involved in the following experimental steps and their preparation methods can be found, for example, in: DNA PROBES G. H. Keller; M. M. Manak; Stockton Press, 1989 (USA) or a more commonly used molecular cloning experimental handbook *Molecular Cloning* (J. Sambrook et al. Acadimic press, 1998, 2nd edition) .

Sample Preparation:

1. DNA Extraction from fresh or frozen tissues

Steps: 1) Place fresh or newly thawed tissue onto a dish on ice containing phosphate-buffered saline (PBS). Cut the tissue into small pieces with scissors or an operating knife. Tissues should be kept damp through the operation. 2) Mince the tissue by centrifugation at 1, 000g for 10 minutes. 3) Re-suspend the pellet (about 10 ml/g) with cold homogenating buffer (0.25 mol/l saccharose; 25 mmol/l Tris-HCl, pH7.5; 25 m mol/LnaCl; 25mmol/L MgCl2) at 4°C, and homogenate tissue suspension at full speed with an electronic homogenizer until it's completely smashed. 5) Centrifuge at 1, 000g for 10 minutes. 6) Re-suspend the cell pellet (1-5 ml per 0.1g initial tissue sample), and centrifuge at 1,000g for 10 minutes. 7) Re-suspend the pellet with lysis buffer (1 ml per 0.1g initial tissue sample), and continue on to the phenol extraction method.

2. Phenol Extraction of DNA

Steps: 1) Wash cells with 1-10ml cold PBS buffer and centrifuge at 1000g for 10 minutes. 2) Re-suspend the precipitated cells with at least 100 µl cold cell lysis buffer (1 108 cells/ml). 3) Add SDS to a final concentration of 1%. Addition of SDS into the cell precipitation before cell re-suspension will cause the formation of large balls by cells which is difficult to be smashed and total production will be reduced. This is especially important when extracting more than $10^7$ cells. 4) Incubate at 50°C for an hour or shake gently overnight at 37°C. 5) Add an equal volume of phenol: chloroform: isoamyl alcohol (25 : 24 : 1) to the DNA solution to be purified in a microcentrifuge tube, and centrifuge for 10 minutes. If the two phases are not clearly separated, the solution should be recentrifuged. 6) Remove the water phase to a new tube. 7) Add an equal volume of chloroform: isoamyl alcohol (24 : 1) and centrifuge for 10 minutes. 8) Remove the water phase containing DNA to a new tube and then purify DNA by ethanol precipitation.

3. DNA Purification By Ethanol Precipitation

Steps: 1) Add 1/10 vol of 2 mol/L sodium acetate and 2 vol of cold 100% ethanol into the DNA solution, mix and place at -20°C for an hour or overnight. 2) Centrifuge for 10 minutes. 3) Carefully remove the ethanol. 4) Add 500 µl of cold 70% ethanol to wash the pellet and centrifuge for 5 minutes. 6) Carefully remove the ethanol and invert the tube on absorbent paper to remove remnant ethanol. Air dry for 10-15 minutes to evaporate the ethanol on the pellet surface. Do not dry the pellet completely since completely dry pellet is difficult to be dissolved again. 7) Re-suspend the DNA pellet with a small volume of TE or water. Spin at low speed or blow with a pipette, and add TE gradually and mix until DNA is completely dissolved. About 1 µl of DNA solution is obtained per $1{\sim}5x10^6$ cells.

The following steps 8-13 are applied only when contamination must be removed, otherwise go directly to step 14. 8) Add Rnase A into DNA solution to a final concentration of 100µg/ml and incubate at 37°C for 30 minutes. 9) Add SDS and protease K to the final concentration of 0.5% and 100µg/ml individually, and incubate at 37°C for 30 minutes. 10) Add an equal volume of phenol: chloroform: isoamyl alcohol (25 : 24 : 1), and centrifuge for 10 minutes. 11) Carefully remove the water phase and extract it with an equal volume of chloroform: isoamyl alcohol (24 : 1) and centrifuge for 10 minutes. 12) Carefully remove out the water phase, and add 1/10 vol of 2mol/L sodium acetate and 2.5 vol of cold 100% ethanol, then mix and place at -20°C for an hour. 13) Wash the pellet with 70% ethanol and 100% ethanol, air dry and re-suspend DNA as same as the steps 3-6. 14) Determine the purity and production of DNA by A260 and A280 assay. 15) Separate DNA sample into several portions and store at -20° C.

Preparation of sample membrane

1) Take 4 2 pieces of nitrocellulose membrane (NC membrane) of desired size, and lightly mark out the sample dot sites and sample number with a pencil. Every probe needs two pieces of NC membrane, so then membranes could be washed under high stringency condition and moderate stringency condition individually in the following experimental steps.

2) Pipette 15 µl of samples and control individually, dot them on the membrane, and dry at room tempreture.

3) Place the membranes on filter paper soaked in 0.1 mol/LnaOH, 1.5 mol/L NaCl, leave for 5 minutes (twice), and allow to dry. Transfer the membranes on filter paper soaked in 0.5 mol/L Tris-HCl (pH7.0), 3mol/L NaCl, leave for 5 minutes (twice), and allow to dry.

4) place the membranes between clean filter paper, packet with aluminum foil, and vacuum dry at 60-80° C for 2 hours.

Labeling of probes

1) Add 3 µl probe (0.1OD/10 l), 2 µl kinase buffer, 8-10 uCi $\gamma$-32P-dATP+2U Kinase, and add water to the final volume of 20 µl.

2) Incubate at 37° C for 2 hours.

3) Add 1/5 vol bromophenol blue indicator (BPB).

4) Load that sample on Sephadex G-50 column.

5) Collect the first peak before the elution of 32P-Probe (monitor the eluting process by Monitor).

6) Five drops each tube and collect for 10-15 tubes.

7) Measure the isotope amount with liquid scintillator.

8) Merged collection of the first peak is the prepared 32P-Probe (the second peak is free γ-32P-dATP).

Prehybridization

Place the sample membranes in a plastic bag, add 3-10mg prehybrid buffer (10×Denhardt, s; 6×SSC, 0.1 mg/ml CT DNA (calf thymus gland DNA)), seal the bag, and shake on a 68° C water bath for two hours hybridization.
Cut off a corner of the plastic bag, add in prepared probes, seal the bag, and shake on a 42° C water bath overnight.

Membrane washing

Membrane washing applying a high- stringency condition:

1) Take out the hybridized sample membranes

2) Wash the membranes with 2×SSC, 0.1% SDS at 40°C for 15 minutes (twice).

3) Wash the membranes with 0.1×SSC, 0.1% SDS at 40°C for 15 minutes (twice).

4) Wash the membranes with 0.1×SSC, 0.1% SDS at 55°C for 30 minutes (twice), and dry at room temperature.

Membrane washing applying a low- stringency condition:

1) Take out the hybridized sample membranes.

2) Wash the membranes with 2×SSC, 0.1% SDS at 37°C for 15 minutes (twice).

3) Wash the membranes with 0.1×SSC, 0.1% SDS at 37°C for 15 minutes (twice).

4) Wash the membranes with 0.1×SSC, 0.1% SDS at 40°C for 15 minutes (twice), and dry at room temperature.

X ray autoradiography:

X ray autoradiograph at -70° C (autoradiograph time varies according to radioactivity of the hybrid spots ).

Experimental results:

In hybridization experiments carried out under low-stringency membrane washing condition, the radioactivity of all the above four probes hybridization spots show no obvious difference; while in hybridization experiments carried out under high-stringency membrane washing condition, radioactivity of the hybrid spot by probe one is obviously stronger than the other three's. So Probe One could be applied in qualitative and quantitative analysis of the existence and differential expression of the polynucleotide of the invention in different tissues.

Industrial Applicability

Polypeptide of the present invention can be quickly phosphorylated by growth factor receptors containing Tyrosine kinase activity. They have no catalytic activity by themselves but can couple activated receptor Tyrosine kinases to other proteins without SH2 and SH3 domain. Human SHC protein 43 of the present invention can regulate the expression of cellular Ras protein and so has important biological functions such as influencing the process of abnormal proliferation of tissue cells and abnormal expression of proteins. So human SHC protein 43 of the present invention can be used for control of cell growth, cell division, cell death, cell differentiation, and other life processes, and further can be applied in diagnosis and cure of corresponding diseases including malignant tumors, cancers, developmental disorders, and immune system diseases.

As the human SHC protein 43 of the present invention is concerned, since expression of the protein is related to genesis of kinds of various malignant tumors and cancers, polypeptide of the present invention can be applied in diagnosis and cure of diseases including the following: gastric carcinoma, hepatocarcinoma, large intestines cancer, mammary cancer, lung cancer, carcinoma of prostate, cancer of uterine cervix, pancreatic cancer; esophageal cancer, pituitary adenoma, thyroid benign tumor, thyroid cancer, parathyroid adenoma, parathyroid cancer, adrenal medulla lipoma, pheochromocytoma, islet cell tumor, multiple endocrine adenopathy, and thymoma.

Human SHC protein 43 of the present invention can also be applied in diagnosis and cure of various corresponding developmental disorders including the following: bifid spine, cranioschisis, anencephadly, encephalocele, schizencephalic porencephaly, Down syndrome, congenital hydrocephalus, Aqueduct deformity, achondroplastic dwarf, spine epiphysis dysplasia, pseudo achondroplasia, Langer-Giedion syndrome, funnel chest, gonadal dysgenesis, Congenital adrenal hyperplasia, epispadia; Anaspadias, deformation syndromes accompanied by Microsomia such as Conradi syndrome and Danbolt-Closs syndrome, congenital glaucoma or cataract, congenital crystalline lens position abnormality, Congenital small palpebral fissure, retina dysplasia, Congenital optic atrophy, Congenital sensory nerve hearing loss, acrorhagadia, teratosis, Willams syndrome, Alagille syndrome, and Bechwith-Wiedemann syndrome.

Human SHC protein 43 of the present invention can also be applied in diagnosis and cure of kinds of various abnormal expression related immune system diseases including the following: proliferating arthritis, chronic active hepatitis, primary xerosis, acute anterior uveitis, gonohemia infection arthritis, ankylosing spondylitis, hemochromatiosis, immue complex-mediated glomerulonephritis, gonohemia infection myocarditis, systemic lupus erythematosus, scleroderma, polymyositis, mouth and eye xerosis, infantile polyarteritis nodosa, Wegener granulomatosis, myasthenia gravis, Guillain-Barre syndrome, autoimmune hemolytic anemia, immunologic thrombocytopenic purpura, autoimmune interstitial nephritis, autoimmune gastritis, insulin autoimmunity syndrome, autoimmune disease of thyroid gland, and autoimmune cardiac disease.

The invention also provides methods for screening compounds so as to identify an agent which enhances human SHC protein 43 activity (agonists) or decrease human SHC protein 43 activity (antagonists). The agonists enhance the biological functions of human SHC protein 43 such as inactivation of cell proliferation, while the antagonists prevent and cure the disorders associated with the excess cell proliferation, such as various cancers. For example, in the presence of an agent, the mammal cells or the membrane preparation expressing human SHC protein 43 can be incubated with the labeled human SHC protein 43 to determine the ability of the agent to enhance or repress the interaction.

Antagonists of human SHC protein 43 include antibodies, compounds, receptor deletants and analogues. The antagonists of human SHC protein 43 can bind to human SHC protein 43 and eliminate or reduce its function, or inhibit the production of human SHC protein 43, or bind to the active site of said polypeptide so that the polypeptide can not function biologically.

When screening for compounds as an antagonist, human SHC protein 43 may be added into a biological assay. It can be determined whether the compound is an antagonist or not by determining its effect on the interaction between human SHC protein 43 and its receptor. Using the same method as that for screening compounds, receptor deletants and analogues acting as antagonists can be selected. Polypeptide molecules capable of binding to human SHC protein 43 can be obtained by screening a polypeptide library comprising various combinations of amino acids bound onto a solid matrix. Usually, human SHC protein 43 is labeled in the screening.

The invention further provides a method for producing antibodies using the polypeptide, and its fragment, derivative, analogue or cells as an antigen. These antibodies may be polyclonal or monoclonal antibodies. The invention also provides antibodies against epitopes of human SHC protein 43. These antibodies include, but are not limited to, polyclonal antibody, monoclonal antibody, chimeric antibody, single-chain antibody, Fab fragment and the fragments produced by a Fab expression library.

Polyclonal antibodies can be prepared by immunizing animals, such as rabbit, mouse, and rat, with human SHC protein 43. Various adjuvants, including but are not limited to Freund's adjuvant, can be used to enhance the immunization. The techniques for producing human SHC protein 43 monoclonal antibodies include, but are not limited to, the hybridoma technique (Kohler and Milstein. Nature, 1975, 256:495-497), the trioma technique, the human B-cell hybridoma technique, the EBV-hybridoma technique and so on. A chimeric antibody comprising a constant region of human origin and a variable region of non-human origin can be produced using methods well-known in the art (Morrison et al, PNAS, 1985, 81:6851). Furthermore, techniques for producing a single-chain antibody (U.S. Patent No. 4946778) are also useful for preparing single-chain antibodies against human SHC protein 43.

The antibody against human SHC protein 43 can be used in immunohistochemical method to detect the presence of human SHC protein 43 in a biopsy specimen.

The monoclonal antibody specific to human SHC protein 43 can be labeled by radioactive isotopes, and injected into human body to trace the location and distribution of human SHC protein 43. This radioactively labeled antibody can be used in the non-wounding diagnostic method for the determination of tumor location and metastasis.

Antibodies can also be designed as an immunotoxin targeting a particular site in the body. For example, a monoclonal antibody having high affinity to human SHC protein 43 can be covalently bound to bacterial or plant toxins, such as diphtheria toxin, ricin, ormosine. One common method is to challenge the amino group on the antibody with sulfydryl cross-linking agents, such as SPDP, and bind the toxin onto the antibody by interchanging the disulfide bonds. This hybrid antibody can be used to kill human SHC protein 43-positive cells.

The antibody of the invention is useful for the therapy or the prophylaxis of disorders related to the human SHC protein 43. The appropriate amount of antibody can be administrated to stimulate or block the production or activity of human SHC protein 43.

The invention further provides diagnostic assays for quantitative and in situ measurement of human SHC protein 43 level. These assays are well known in the art and include FISH assay and radioimmunoassay. The level of human SHC protein 43 detected in the assay can be used to illustrate the importance of human SHC protein 43 in diseases and to determine the diseases associated with human SHC protein 43.

The polypeptide of the invention is useful in the analysis of polypeptide profile. For example, the polypeptide can be specifically digested by physical, chemical, or enzymatic means, and then analyzed by one, two or three dimensional gel electrophoresis, preferably by spectrometry.

New human SHC protein 43 polynucleotides also have many therapeutic applications. Gene therapy technology can be used in the therapy of abnormal cell proliferation, development or metabolism, which are caused by the loss of human SHC protein 43 expression or the abnormal or non-active expression of human SHC protein 43. Recombinant gene therapy vectors, such as virus vectors, can be designed to express mutated human SHC protein 43 so as to inhibit the activity of endogenous human SHC protein 43. For example, one form of mutated human SHC protein 43 is a truncated human SHC protein 43 whose signal transduction domain is deleted. Therefore, this mutated human SHC protein 43 can bind the downstream substrate without the activity of signal transduction. Thus, the recombinant gene therapy vectors can be used to cure diseases caused by abnormal expression or activity of human SHC protein 43. The expression vectors derived from a virus, such as retrovirus, adenovirus, adeno-associated virus, herpes simplex virus, parvovirus, and so on, can be used to introduce the human SHC protein 43 gene into the cells. The methods for constructing a recombinant virus vector harboring human SHC protein 43 gene are described in the literature (Sambrook, et al. supra). In addition, the recombinant human SHC protein 43 gene can be packed into liposome and then transferred into the cells.

The methods for introducing the polynucleotides into tissues or cells include directly injecting the polynucleotides into tissue in the body; or introducing the polynucleotides into cells in vitro with vectors, such as virus, phage, or plasmid, etc, and then transplanting the cells into the body.

Also included in the invention are ribozyme and the oligonucleotides, including antisense RNA and DNA, which inhibit the translation of the human SHC protein 43 mRNA. Ribozyme is an enzyme-like RNA molecule capable of specifically cutting certain RNA. The mechanism is nucleic acid endo-cleavage following specific hybridization of ribozyme molecule and the complementary target RNA. Antisense RNA and DNA as well as ribozyme can be prepared by using any conventional techniques for RNA and DNA synthesis, e.g., the widely used solid phase phosphite chemical method for oligonucleotide synthesis. Antisense RNA molecule can be obtained by the in vivo or in vitro transcription of the DNA sequence encoding said RNA, wherein said DNA sequence is integrated into the vector and downstream of the RNA polymerase promoter. In order to increase its stability, a nucleic acid molecule can be modified in many manners, e.g., increasing the length of two the flanking sequences, replacing the phosphodiester bond with the phosphothioester bond in the oligonucleotide.

The polynucleotide encoding human SHC protein 43 can be used in the diagnosis of human SHC protein 43 related diseases. The polynucleotide encoding human SHC protein 43 can be used to detect whether human SHC protein 43 is expressed or not, and whether the expression of human SHC protein 43 is normal or abnormal in the case of diseases. For example, human SHC protein 43 DNA sequences can be used in the hybridization with biopsy samples to determine the expression of human SHC protein 43. The hybridization methods include Southern blotting, Northern blotting and in situ blotting, etc., which are well-known and established techniques. The corresponding kits are commercially available. A part of or all of the polynucleotides of the invention can be used as probe and fixed on a microarray or DNA chip for analysis of differential expression of genes in tissues and for the diagnosis of genes. The human SHC protein 43 specific primers can be used in RNA-polymerase chain reaction and in vitro amplification to detect transcripts of human SHC protein 43.

Further, detection of mutations in human SHC protein 43 gene is useful for the diagnosis of human SHC protein 43 -related diseases. Mutations of human SHC protein 43 include site mutation, translocation, deletion, rearrangement and any other mutations compared with the wild-type human SHC protein 43 DNA sequence. The conventional methods, such as Southern blotting, DNA sequencing, PCR and in situ blotting, can be used to detect a mutation. Moreover, mutations sometimes affects the expression of protein. Therefore, Northern blotting and Western blotting can be used to indirectly determine whether the gene is mutated or not.

Sequences of the present invention are also valuable for chromosome identification. The sequence is specifically targeted to and can hybridize with a particular location on an individual human chromosome. There is a current need for identifying particular sites of gene on the chromosome. Few chromosome marking reagents based on actual sequence data (repeat polymorphism) are presently available for marking chromosomal location. The mapping of DNA to chromosomes according to the present invention is an important first step in correlating those sequences with genes associated with disease.

Briefly, sequences can be mapped to chromosomes by preparing PCR primers (preferably 15-35 bp) from the cDNA. These primers are then used for PCR screening of somatic cell hybrids containing individual human chromosomes. Only those hybrids containing the human gene corresponding to the primer will yield an amplified fragment.

PCR mapping of somatic cell hybrids is a rapid procedure for assigning a particular DNA to a particular chromosome. Using the oligonucleotide primers of the invention, sublocalization can be achieved with panels of fragments from specific chromosomes or pools of large genomic clones in an analogous manner. Other mapping strategies that can similarly be used to map to its chromosome include in situ hybridization, prescreening with labeled flow-sorted chromosomes and preselection by hybridization to construct chromosome specific-cDNA libraries.

Fluorescence in situ hybridization (FISH) of a cDNA clones to a metaphase chromosomal spread can be used to provide a precise chromosomal location in one step. For a review of this technique, see Verma et al., Human Chromosomes: a Manual of Basic Techniques, Pergamon Press, New York (1988).

Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis.

Next, it is necessary to determine the differences in the cDNA or genomic sequence between affected and unaffected individuals. If a mutation is observed in some or all of the affected individuals but not in any normal individuals, then the mutation is likely to be the cause of the disease. Comparison of affected and unaffected individuals generally involves first looking for structural alterations in the chromosomes, such as deletions or translocations, that are visible from chromosome level, or detectable using PCR based on that DNA sequence. With current resolution of physical mapping and genetic mapping techniques, a cDNA precisely localized to a chromosomal region associated with the disease could be one of 50 to 500 potential causative genes. (This assumes 1 megabase mapping resolution and one gene per 20 kb).

According to the invention, the polypeptides, polynucleotides and its mimetics, agonists, antagonists and inhibitors may be employed in combination with a suitable pharmaceutical carrier. Such a carrier includes but is not limited to water, glucose, ethanol, salt, buffer, glycerol, and combinations thereof. Such compositions comprise a safe and effective amount of the polypeptide or antagonist, as well as a pharmaceutically acceptable carrier or excipient with no influence on the effect of the drug. These compositions can be used as drugs in disease treatment.

The invention also provides a pharmaceutical pack or kit comprising one or more containers filled with one or more of the ingredients of the pharmaceutical compositions of the invention. With such container(s) there may be a notice from a governmental agency, that regulates the manufacture, use or sale of pharmaceuticals or biological products, the notice reflects government's approval for the manufacture, use or sale for human administration. In addition, the polypeptides of the invention may be employed in conjunction with other therapeutic compounds.

The pharmaceutical compositions may be administered in a convenient manner, such as through topical, intravenous, intraperitoneal, intramuscular, subcutaneous, intranasal or intradermal routes. human SHC protein 43 is administered in an amount, which is effective for treating and/or prophylaxis of the specific indication. The amount of human SHC protein 43 administered on patient will depend upon various factors, such as delivery methods, the subject health, the judgment of the skilled clinician.

**Claims**

1. An isolated polypeptide -human SHC protein 43- comprising a polypeptide having the amino acid sequence of SEQ ID NO: 2, its active fragments, analogues and derivatives.

2. The polypeptide of Claim 1 wherein amino acid sequences of said polypeptide, its analogues or derivatives have at least 95% identity with the amino acid sequence of SEQ ID NO: 2.

3. The polypeptide of Claim 2 wherein said polypeptide is a polypeptide comprising the amino acid sequence of SEQ ID NO: 2.

4. An isolated polynucleotide selected from the group consisting of:

   (a) the polynucleotide encoding a polypeptide having an amino acid sequence of SEQ ID NO: 2 or its fragment, analogue, derivative;

   (b) the polynucleotide complementary to polynucleotide (a); and

   (c) the polynucleotide sharing at least 70% identity to polynucleotide (a) or (b).

5. The polynucleotide of claim 4 comprising a polynucleotide encoding an amino acid sequence of SEQ ID NO:2.

6. The polynucleotide of claim 4 wherein the sequence of said polynucleotide comprises position 996-2159 of SEQ ID NO:1 or position 1-2719 of SEQ ID NO:1.

7. A recombinant vector containing an exogenous polynucleotide which is constructed with the polynucleotide of any of claims 4-6 and plasmid, virus, or expression vector.

8. A genetically engineered host cell containing an exogenous polynucleotide which is selected form the group consisting of:

   (a) the host cell transformed or transfected by the recombinant vector of claim 7; and

   (b) the host cell transformed or transfected by the polynucleotide of any of claims 4-6.

9. A method for producing a polypeptide having the activity of human SHC protein 43, which comprises the steps of:

   (a) culturing the engineered host cell of claim 8 under the conditions suitable for expression of human SHC protein 43;

   (b) isolating the polypeptides having the activity of human SHC protein 43 protein from the culture.

10. An antibody specifically which binds bound specifically with human SHC protein 43.

11. A compound simulating or regulating the activity or expression of the polypeptide which is the compound simulating, improving, antagonizing, or inhibiting the activity of human SHC protein 43.

12. The compound of claim 11 which is an antisense sequence of the polynucleotide sequence of SEQ ID NO: 1 or its fragment.

13. The use of the compound of claim 11 for regulating the activity of human SHC protein 43 *in vivo* or *in vitro.*

14. A method for detecting a disease related to the polypeptide of any of claims 1-3 or susceptibility thereof which comprises detecting the amount of expression of said polypeptide, or detecting the activity of said polypeptide, or detecting the nucleotide variant of the polynucleotide causing said abnormal expression or activity.

15. The use of the polypeptide of any of claims 1-3 for screening the mimetics, agonists, antagonists or inhibitors of human SHC protein 43; or for the identification of peptide spectrum.

16. The use of the nucleic acid molecule of any of claims 4-6 wherein it is used as primer in the nucleic acid amplification, or as probe in the hybridization reaction, or is used for manufacture of gene chip or microarray.

17. The use of the polypeptide, polynucleotide or compound of any of claims 1-6 and 11 wherein a safe and effective amount of said polypeptide, polynucleotide or its mimetics, agonists, antagonists or inhibitors are mixed with the pharmaceutically acceptable carrier to form the pharmaceutical composition for the diagnosis or treatment of diseases associated with the abnormality of human SHC protein 43.

18. The use of the polypeptide, polynucleotide or compound of any of claims 1-6 and 11 wherein said polypeptide, polynucleotide or compound are used for the manufacture of medicine for the treatment of developmental disorders, diseases caused by abnormal metabolism of immune system, and cancers.

**Identity = 54%, Similarity = 65%**

```
Query:  1098 CDHIIANHHMQSISFASGGDPDTTDYVAYVAKDPVNQRACHILECHNGMAQDVISTIGQA 1277
             C  IIANHHMQSISFASGGDPDT +YVAYVAKDPVNQRACHILEC  G+AQDVISTIGQA
Sbjct:   138 CKQIIANHHMQSISFASGGDPDTAEYVAYVAKDPVNQRACHILECPEGLAQDVISTIGQA 197


Query:  1278 FELRFKQYLKNPSLNTSCESEEVHIDSHA---EERE--DHEYYNEIPGKQPPVGGVSDMR 1442
             FELRFKQYL+NP    +        D A   EE E  DH+YYN+ PGK+PP+GGV DMR
Sbjct:   198 FELRFKQYLRNPPKLVTPHDRMAGFDGSAWDEEEEEPPDHQYYNDFPGKEPPLGGVVDMR 257


Query:  1443 IKVQATEQMAYCPIQCEKLCYLPGNSKCSSVYENCLEQSRAIGNVHPRGVQSQRDTSLLK 1622
             ++  A   A  P        P N++  S       L    + +G   P  V+ Q
Sbjct:   258 LREGAAPGAAR-PTA-------P-NAQTPSHLGATLPVGQPVGG-DPE-VRKQMPPP--- 303


Query:  1623 HTCRV-DLFDDPCYINTQALQSTPGSAGNQRSAQPLGSPWHCGKAPETV---QP--GATA 1784
             C   +LFDDP Y+N Q L     + G   +  P +P   G AP +   +P   A
Sbjct:   304 PPCPGRELFDDPSYVNVQNLDKARQAVGG--AGPP--NPAINGSAPRDLFDMKPFEDALR 359


Query:  1785 QPASSHSLPHIKQQLWSEECYHGKLSRKAAESLLVKDGDFLVRESATSPGQYVLSGLQGG 1964
             P   S+  + +QL  E  +HGKLSR+ AE+LL   +GDFLVRES T+PGQYVL+GLQ G
Sbjct:   360 VPPPPQSVS-MAEQLRGEPWFHGKLSRREAEALLQLNGDFLVRESTTTPGQYVLTGLQSG 418


Query:  1965 QAKHLLLVDPEGKVRTKDHVFDNVGHLIRYHMDNSLPIISSGSEVSLKQPVRK 2123
             Q KHLLLVDPEG VRTKDH F++V HLI YHMDN LPIIS+GSE+ L+QPV +
Sbjct:   419 QPKHLLLVDPEGVVRTKDHRFESVSHLISYHMDNHLPIISAGSELCLQQPVER 471
```

Query: Human SHC protein 43; Sbjct: The early found human SHC protein

FIG. 1

FIG. 2

**EP 1 251 178 A1**

INTERNATIONAL SEARCH REPORT

International application No.

PCT/CN00/00589

**A.**  CLASSIFICATION OF SUBJECT MATTER

$IPC^7$ C12N15/57, C07K14/745

According to International Patent Classification(IPC) or to both national classification and IPC

**B.**  FIELDS SEARCHED

Minimum documentation searched(classification system followed by classification symbols)

$IPC^7$ C12N15/57, C07K14/745

Documentation searched other than minimum documentation to the extent that such documents are included in the field searched

Electronic data base consulted during the international search(name of data base and, where practicable, search terms used)

CNPAT, EPOQUE(WPI), NCBI

**C.**  DOCUMENTS CONSIDERED TO BE RELEVANT

| Category* | Citation of document, with indication, where appropriate, of the relevant passages | Relevant claim No. |
|---|---|---|
| A | GenBank Accession NO: GI: 4249548(Kim, Y.B. et al) 10.02.1999, See the Abstract | 1-13,15-18 |
| A | GenBank Accession NO: GI: 284403(Pelicci, G. et al) 05.11.1999, See the Abstract | 1-13,15-18 |

☐  Further documents are listed in the continuation of Box C.       ☐  See patent family annex.

| | |
|---|---|
| *    Special categories of cited documents:<br>"A"  document defining the general state of the art which is not considered to be of particular relevance<br>"E"  earlier document but published on or after the international filing date<br>"L"  document which may throw doubts on priority claim(s) or which is cited to establish the publication date of another citation or other special reason(as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |
| Date of the actual completion of the international search<br><br>12 March 2001(12.03.2001) | Date of mailing of the international search report<br><br>29 MAR 2001 |
| Name and mailing address of the ISA/<br>The Chinese Patent Office<br>6, Xitucheng Road, Haidian District,<br>Beijing, 100088, China<br>Facsimile No.        86-10-62019451 | Authorized officer<br><br>PAN, aiqun<br><br>Telephone No.    86-10-62093906 |

Form PCT/ISA/210(second sheet)(July 1992)

25

SEQUENCE LISTING

(1)   General information:
      (i)    Title of invention:    HUMAN SHC PROTEIN 43 AND THE POLYNUCLEOTIDE
                                    ENCODING THE POLYPEPTIDE

      (ii)   Number of Sequences: 9

(2)   INFORMATION FOR SEQ ID NO: 1:
      (i)    SEQUENCE CHARACTERISTICS:
             (A)    LENGTH: 2719bp
             (B)    TYPE: nucleic acid
             (C)    STRANDEDNESS: double
             (D)    TOPOLOGY: linear
      (ii)   MOLECULAR TYPE: cDNA
      (xi)   SEQUENCE DESCRIPTION: SEQ ID NO: 1:

```
   1 GTACTTTTTTTTTTTAGTTATAGTGAAGAAATACTTGGTCCCATTTCTTTTCGCCTGGGA
  61 GGTATCTTTCCACTATTAGCAATGCGGTGTGTATTCTTTGAATTCTTTTTATTTGCATTT
 121 ACATGTATCTTTATGTATTGACAGCAAAATACTATTTTGTGTGTTTTTTAAAAACATGTT
 181 TTACCTGTTTTTACCTTCAACTTAAAAGCCTCAGGTTTTAAGTTGTATATACTTATATGA
 241 ATTATATGACTGTGTATACTTACAGGATTCAAGCACATTTCATTTATTCTGCACTTTATT
 301 TCTATTATTATTACATTATAGTTATGTAGAATTATACAACTCACCATAATGTAGAATCAA
 361 TGGGAGCCCTGAGCTTGTTTTCCTGCAACTAGATGGTCCCATCTGGGGGTGATGAGACAC
 421 AGTGACACCCGAGGTGTGTTCCTTATGTCCGGTCTATTCTGTAATCTCATTTTGGTTGCT
 481 GTTACTGTGGAAAATCCTGCCTCACAAAGATAGGATGTTGAAAATGGAAGGAGGCTTTTC
 541 AGTACTTTTGTGGCAATCTCAGGATGTTCCGCCTTGACTTTAATCGAGAACGTATGGAGA
 601 TTTGAAGTTGTCTCAAACATACTTTTAAGGCCACCGTCATTTGCAATATCAAGCAGTTGA
 661 TCCTCTTCTAGCGTGGACAAAGTGGATTCACCTGGCTTATTCACAAATGGGTCACAGATC
 721 CATTCCGTCTTTTGTGGATGAGAAGTGTGATGGTTAATACTGAGTGTCAACTTGATTGGA
 781 TTGAAGGATACAAGTATTGATCATTGGTGTGTCTGTGAGGGTGTTGCCAAAGGAGATTAA
 841 CATTTGAGTCAGTGGGCTGCGAAAGGCAGACCCACCCTTAATCTGGTTGGGCACAATCTA
 901 ATCAGCTGCCAGCTTGGCTAGAATATAAGCAGGCAGAAAAATGTGAAAAGAGAGACTGGC
 961 CTAGCCTCCCAGCCTAAATCTTTCTCCTGTGTTGGATGCTTCCTGCCCTCGAACATTGGA
1021 TTCCCAAGTTCTTCAGTTTCAGAACTCGGACTGGCTCTCCTCTCTCCTTAGCCTGCAGAC
1081 AGCCTATTGTAGGACCTTGTGATCATATTATTGCAAATCATCATATGCAGTCTATTTCAT
1141 TTGCCTCTGGAGGGGATCCTGATACTACAGACTATGTTGCCTACGTAGCTAAAGATCCAG
1201 TTAATCAACGAGCCTGTCACATATTGGAATGCCACAATGGAATGGCCCAAGACGTCATAA
1261 GTACCATAGGGCAGGCTTTTGAACTCCGGTTTAAACAGTACTTGAAAAATCCTTCTTTGA
1321 ATACTTCTTGTGAAAGTGAGGAGGTGCATATTGATAGCCATGCCGAGGAGAGAGAAGATC
1381 ATGAATATTACAATGAAATTCCAGGGAAGCAGCCACCAGTAGGTGGTGTTTCAGATATGC
1441 GGATCAAAGTTCAAGCCACGGAACAAATGGCTTACTGCCCCATACAGTGTGAAAAGTTGT
```

```
1501 GCTATTTGCCTGGAAACTCCAAGTGCAGCAGTGTATATGAGAACTGTTTAGAACAAAGCA
1561 GGGCAATAGGTAATGTCCATCCAAGAGGGGTGCAGTCCCAGCGAGATACCTCATTATTGA
1621 AGCACACGTGCCGAGTGGATCTCTTTGATGACCCCTGCTACATTAATACACAGGCTCTTC
1681 AAAGTACACCTGGCTCTGCTGGAAATCAAAGGTCAGCCCAACCACTGGGGAGCCCATGGC
1741 ACTGCGGAAAGGCACCAGAAACTGTTCAGCCGGGTGCCACAGCCCAGCCTGCCAGCTCAC
1801 ATTCTTTGCCACACATTAAGCAGCAGCTGTGGAGCGAAGAATGCTATCATGGCAAGCTGA
1861 GCAGGAAGGCGGCAGAGAGCCTCTTGGTAAAGGATGGGGACTTTTTGGTTCGAGAGAGTG
1921 CAACATCCCCTGGCCAATATGTGCTGAGTGGACTACAGGGAGGCCAAGCAAAACATCTTC
1981 TCCTGGTGGATCCTGAAGGCAAGGTGAGGACCAAGGATCATGTATTTGATAATGTCGGCC
2041 ACCTTATCAGATACCATATGGATAACAGTTTGCCAATCATCTCCTCTGGAAGCGAAGTAA
2101 GCCTTAAACAACCAGTGAGAAAAGATAATAATCCAGCACTTTTGCATTCCAACAAATGAC
2161 AGTATTGAAGCACCATCACACTGATATTTCAAGAAACCCCATTTTGTATTAGGACACAAA
2221 GATAATTTAAACTTTGTTTGTAGATAAAATAGAGCACAAACTGTGAAGTGCATCTTTCCA
2281 AGACCATCATGGACCAGGTCCTCTATAAAATGAAGAACTAACAAAAATTAGTCTTCAGAA
2341 ATGAAAATCAGAAAAGAGGAAGAGGGTTGGTCATTTTAAAAGAAATTATATGTATGCACG
2401 GATGTCACTTTTTAAGGCCATATTGCATTGATAACAAGCTAAAAGCACAACTAAAATTTC
2461 ACATGCTAACGACAACTTGAATGAACTGCTGGGGCAGTGGTATGTGCCTTTCAACTTGAT
2521 AATTTGGGGGACATTTTCATATTGGGAGATTAATTCTAAGTATCTTCATGTTCTATGACT
2581 ATAGAACCATTTGCCAAAAAAAAAAGCTTTTCTTGCTACAAAAAATAAGCAATTTTCTTG
2641 AGCCTTATTGACTTTATTACATTTTCTGTTTAGCAGCATTTTTCACTGCAATGTTAAAAT
2701 AAATATGACATTGAATTCG
```

(3)    INFORMATION FOR SEQ ID NO: 2:
        (i)    SEQUENCE CHARACTERISTICS:
                (A)    LENGTH: 387 amino acids
                (B)    TYPE: amino acid
                (D)    TOPOLOGY: lineal
        (ii)   MOLECULE TYPE: polypeptide
        (xi)   SEQUENCE DESCRIPTION: SEQ ID NO: 2:

```
  1 Met Leu Pro Ala Leu Glu His Trp Ile Pro Lys Phe Phe Ser Phe
 16 Arg Thr Arg Thr Gly Ser Pro Leu Ser Leu Ala Cys Arg Gln Pro
 31 Ile Val Gly Pro Cys Asp His Ile Ile Ala Asn His His Met Gln
 46 Ser Ile Ser Phe Ala Ser Gly Gly Asp Pro Asp Thr Thr Asp Tyr
 61 Val Ala Tyr Val Ala Lys Asp Pro Val Asn Gln Arg Ala Cys His
 76 Ile Leu Glu Cys His Asn Gly Met Ala Gln Asp Val Ile Ser Thr
 91 Ile Gly Gln Ala Phe Glu Leu Arg Phe Lys Gln Tyr Leu Lys Asn
106 Pro Ser Leu Asn Thr Ser Cys Glu Ser Glu Glu Val His Ile Asp
121 Ser His Ala Glu Glu Arg Glu Asp His Glu Tyr Tyr Asn Glu Ile
136 Pro Gly Lys Gln Pro Pro Val Gly Gly Val Ser Asp Met Arg Ile
151 Lys Val Gln Ala Thr Glu Gln Met Ala Tyr Cys Pro Ile Gln Cys
166 Glu Lys Leu Cys Tyr Leu Pro Gly Asn Ser Lys Cys Ser Ser Val
```

18

```
181 Tyr Glu Asn Cys Leu Glu Gln Ser Arg Ala Ile Gly Asn Val His
196 Pro Arg Gly Val Gln Ser Gln Arg Asp Thr Ser Leu Leu Lys His
211 Thr Cys Arg Val Asp Leu Phe Asp Asp Pro Cys Tyr Ile Asn Thr
226 Gln Ala Leu Gln Ser Thr Pro Gly Ser Ala Gly Asn Gln Arg Ser
241 Ala Gln Pro Leu Gly Ser Pro Trp His Cys Gly Lys Ala Pro Glu
256 Thr Val Gln Pro Gly Ala Thr Ala Gln Pro Ala Ser Ser His Ser
271 Leu Pro His Ile Lys Gln Gln Leu Trp Ser Glu Glu Cys Tyr His
286 Gly Lys Leu Ser Arg Lys Ala Ala Glu Ser Leu Leu Val Lys Asp
301 Gly Asp Phe Leu Val Arg Glu Ser Ala Thr Ser Pro Gly Gln Tyr
316 Val Leu Ser Gly Leu Gln Gly Gly Gln Ala Lys His Leu Leu Leu
331 Val Asp Pro Glu Gly Lys Val Arg Thr Lys Asp His Val Phe Asp
346 Asn Val Gly His Leu Ile Arg Tyr His Met Asp Asn Ser Leu Pro
361 Ile Ile Ser Ser Gly Ser Glu Val Ser Leu Lys Gln Pro Val Arg
376 Lys Asp Asn Asn Pro Ala Leu Leu His Ser Asn Lys
```

(4) INFORMATION FOR SEQ ID NO: 3:

    (i)    SEQUENCE CHARACTERISTICS:

        (A)    LENGTH: 24bp

        (B)    TYPE: nucleic acid

        (C)    STRANDEDNESS: single

        (D)    TOPOLOGY: linear

    (ii)    MOLECULAR TYPE: oligonucleotide

    (xi)    SEQUENCE DESCRIPTION: SEQ ID NO: 3:

GTACTTTTTTTTTTTAGTTATAGT        24


(5) INFORMATION FOR SEQ ID NO: 4:

    (i)    SEQUENCE CHARACTERISTICS:

        (A)    LENGTH: 24bp

        (B)    TYPE: nucleic acid

        (C)    STRANDEDNESS: single

        (D)    TOPOLOGY: linear

    (ii)    MOLECULAR TYPE: oligonucleotide

    (xi)    SEQUENCE DESCRIPTION: SEQ ID NO: 4:

CGAATTCAATGTCATATTTATTTT        24


(6) INFORMATION FOR SEQ ID NO: 5:

    (i)    SEQUENCE CHARACTERISTICS:

        (A)    LENGTH: 34bp

        (B)    TYPE: nucleic acid

        (C)    STRANDEDNESS: single

        (D)    TOPOLOGY: linear

    (ii)    MOLECULAR TYPE: oligonucleotide

      (xi)   SEQUENCE DESCRIPTION: SEQ ID NO: 5:

CATGCTAGCATGCTTCCTGCCCTCGAACATTGGA                  34

(7)   INFORMATION FOR SEQ ID NO: 6:

    (i)   SEQUENCE CHARACTERISTICS:

        (A)   LENGTH:34bp

        (B)   TYPE: nucleic acid

        (C)   STRANDEDNESS: single

        (D)   TOPOLOGY: linear

    (ii)  MOLECULAR TYPE: oligonucleotide

    (xi)  SEQUENCE DESCRIPTION: SEQ ID NO: 6:

CCCGAGCTCTCATTTGTTGGAATGCAAAAGTGCT                  34

(8)   INFORMATION FOR SEQ ID NO: 7:

    (i)   SEQUENCE CHARACTERISTICS:

        (A)   LENGTH: 15 amino acids

        (B)   TYPE: amino acid

        (D)   TOPOLOGY: lineal

    (ii)  MOLECULE TYPE: polypeptide

    (xi)  SEQUENCE DESCRIPTION: SEQ ID NO: 7:

Met-Leu-Pro-Ala-Leu-Glu-His-Trp-Ile-Pro-Lys-Phe-Phe-Ser-Phe     15

(9)   INFORMATION FOR SEQ ID NO: 8:

    (i)   SEQUENCE CHARACTERISTICS:

        (A)   LENGTH: 41 bp

        (B)   TYPE: nucleic acid

        (C)   STRANDEDNESS: single

        (D)   TOPOLOGY: lineal

    (ii)  MOLECULE TYPE: oligonucleotide

    (xi)  SEQUENCE DESCRIPTION: SEQ ID NO: 4:

CCAAGTTCTTCAGTTTCAGAACTCGGACTGGCTCTCCTCTC          41

(10)  INFORMATION FOR SEQ ID NO: 8:

    (i)   SEQUENCE CHARACTERISTICS:

        (A)   LENGTH: 41 bp

        (C)   TYPE: nucleic acid

        (C)   STRANDEDNESS: single

        (D)   TOPOLOGY: lineal

    (ii)  MOLECULE TYPE: oligonucleotide

    (xi)  SEQUENCE DESCRIPTION: SEQ ID NO: 9:

CCAAATCGTTCAGTTTCAGATCGCGGAATCGCTCTATCGTC          41